Europäisches Patentamt

European Patent Office

Office européen des brevets

(19)

(11) Numéro de publication : **0 497 648 A1**

(12) **DEMANDE DE BREVET EUROPEEN**

(21) Numéro de dépôt : **92400125.8**

(22) Date de dépôt : **17.01.92**

(51) Int. Cl.⁵ : **A61B 5/16, A61B 3/02**

(30) Priorité : **29.01.91 FR 9100965**

(43) Date de publication de la demande :
**05.08.92 Bulletin 92/32**

(84) Etats contractants désignés :
**DE ES FR GB IT**

(71) Demandeur : **BUCHMANN OPTICAL
ENGINEERING, Société dite:
Rozendaalstraat 14
B-8900 Ieper (BE)**

(72) Inventeur : **Neetens, Adolphe
Solhofdreef 66
B-2630 Aartselaar (BE)**
Inventeur : **van den Ende, Madame Patricia
Tentoonstellingslaan 438 W3
B-1090 Brussel (BE)**

(74) Mandataire : **Barnay, André François
Cabinet Barnay 80 rue Saint-Lazare
F-75009 Paris (FR)**

(54) **Appareil pour la mesure de la fréquence critique de fusion fovéale de l'oeil.**

(57)    L'appareil selon l'invention permet la mesure de la fréquence de fusion critique de la fovéa de l'oeil d'un individu grâce à une source lumineuse intermittente ponctuelle (88) qui peut émettre dans deux longueurs d'ondes distinctes correspondant à deux couleurs distinctes perceptibles par l'individu. La source lumineuse (88) est sensiblement ponctuelle et il est prévu des moyens de réglage (54, 110) de la position relative des oculaires (48, 50, 52) de manière à adapter l'appareil à l'écart pupillaire de l'individu.

FIG.2

EP 0 497 648 A1

La présente invention concerne un appareil pour la mesure de la fréquence critique de fusion fovéale de l'oeil d'un individu.

On appelle fréquence de fusion de l'oeil, la fréquence de clignotement d'une source lumineuse intermittente au delà de laquelle l'oeil de l'individu perçoit la source lumineuse comme étant une source lumineuse à émission continue et non plus intermittente.

La détermination de la fréquence de fusion de l'oeil est particulièrement importante pour établir un diagnostic et évaluer l'acuité visuelle d'un individu.

Divers appareils pour la mesure de la fréquence de fusion de l'oeil ont déjà été proposés et sont couramment utilisés.

Chacun de ces appareils comporte au moins un oculaire auquel l'individu applique son oeil, une source lumineuse intérmittente visible par l'individu à travers l'oculaire, des moyens pour faire varier la fréquence de clignotement de la source lumineuse et des moyens d'enregistrement de la fréquence d'émission de la source lumineuse, ainsi que des moyens de commande des moyens d'enregistrement qui sont déclenchés par l'individu lorsque la fréquence de fusion de l'oeil est atteinte.

Les différentes versions des appareils existants ont eu principalement pour but de maîtriser les différents paramètres susceptibles d'avoir une influence sur la fréquence de fusion mesurée, tels que notamment l'intensité de la lumière, la durée des impulsions lumineuses, la surface de la zone stimulée de l'oeil, l'homogénéité lumineuse de l'arrière par rapport auquel on observe la source intermittente, et la vision binoculaire optimale.

Le document WO 88/10088 décrit et représente un appareil dont la source lumineuse est une lampe plasmatique incandescente associée à une série de filtres de couleurs différentes qui permettent d'émettre selon plusieurs longueurs d'onde.

Cet appareil de mesure ne permet pas de maîtriser de manière fiable et précise certains des paramètres qui ont une influence lors de la mesure de la fréquence de fusion, et notamment la longueur d'onde émise, l'intensité de l'éclairement, etc.

Cet appareil ne permet donc pas non plus d'éliminer les erreurs de mesure dues à des disfonctionnements de l'oeil dans la perception des couleurs.

Afin de remédier à ces inconvénients, l'invention propose un appareil pour la mesure de la fréquence critique de fusion fovéale de l'oeil d'un individu, du type comportant :

- au moins un oculaire auquel l'individu applique son oeil;
- une source lumineuse intermittente visible par l'individu à travers l'oculaire;
- des moyens pour faire varier la fréquence d'émission de la source lumineuse intermittente;
- des moyens d'enregistrement de la fréquence d'émission de la source lumineuse;
- des moyens de commande des moyens d'enregistrement déclenchés par l'individu lorsque la fréquence de fusion de l'oeil est atteinte; et
- une source lumineuse susceptible d'émettre selon plusieurs longueurs d'onde;

caractérisé en ce que la source lumineuse émet selon au moins deux longueurs d'onde constantes correspondant à deux couleurs distinctes perceptibles par l'individu, et en ce que l'appareil comporte des mayens pour provoquer l'émission variable en fréquence de la source lumineuse aux deux longueurs d'ondes distinctes afin de mesurer successivement la fréquence critique de fusion fovéale de l'oeil de l'individu pour les deux longueurs d'ondes.

La source lumineuse comporte deux diodes électro-luminescentes.

Les deux couleurs distinctes d'émission de la source lumineuse sont de préférence le rouge et le vert.

Les moyens pour faire varier la fréquence permettent d'augmenter ou de réduire progressivement, selon un pas constant, la fréquence d'émission lumineuse pour chacune des deux longueurs d'ondes.

Selon d'autres caractéristiques de l'appareil :

- il comporte un circuit électronique pour alimenter là source lumineuse de manière que l'intensité de la lumière émise soit maintenue constante et qu'elle émette de façon intermittente avec des impulsions de durée constante au cours de la mesure;
- il comporte des moyens pour produire un fond illuminé homogène en arrière de la source lumineuse et perceptible par l'individu;
- il comporte un deuxième oculaire et la source lumineuse est visible par l'individu à travers un seul des deux oculaires.

L'invention concerne plus particulièrement un appareil pour la mesure de la fréquence de fusion de la fovéa de l'oeil d'un individu.

La fovéa est une dépression de la partie de la rétine qui est située dans l'axe optique de l'oeil (aire centrale), au centre de la "tache jaune". L'acuité visuelle de l'homme est améliorée par une plus forte densité et une répartition différente des cellules photoréceptrices de l'aire centrale et ceci notamment du fait de l'existence de la fovéa dont les cellules visuelles sont reliées chacune à un axone différent du tractus optique (nerf optique).

Afin de mesurer la fréquence de fusion de la fovéa de l'oeil, l'appareil selon l'invention comporte une source lumineuse qui est sensiblement ponctuelle et il comporte des moyens de réglage de la position de l'oeil mesuré par rapport à la source lumineuse intermittente.

La description qui va suivre, en regard des dessins annexés à titre d'exemples non limitatifs, permettra de bien comprendre comment l'invention peut être mise en pratique.

La figure 1 est une vue en perspective d'un mode de réalisation de l'appareil selon l'invention.

La figure 2 est une vue de dessus de l'appareil de la figure 1 dont on a ôté le couvercle de fermeture.

La figure 3 est une vue de détail en coupe partielle illustrant le dispositif de réglage des oculaires.

La figure 4 est une vue du dispositif de la figure 3 selon la flèche F de cette figure.

Les figures 5 à 8 sont des diagrammes illustrant des résultats de mesures effectuées à l'aide de l'appareil illustré aux figures 1 à 4.

La figure 9 est une vue de dessus d'un mode de réalisation préféré d'un appareil de mesure selon l'invention.

Les figures 10 à 12 sont des vues latérales et en élévation de l'appareil illustré à la figure 9.

Les figures 13 à 15 sont des vues de détails en coupe partielle selon les lignes de coupe correspondantes indiquées sur les figures 9 et 14.

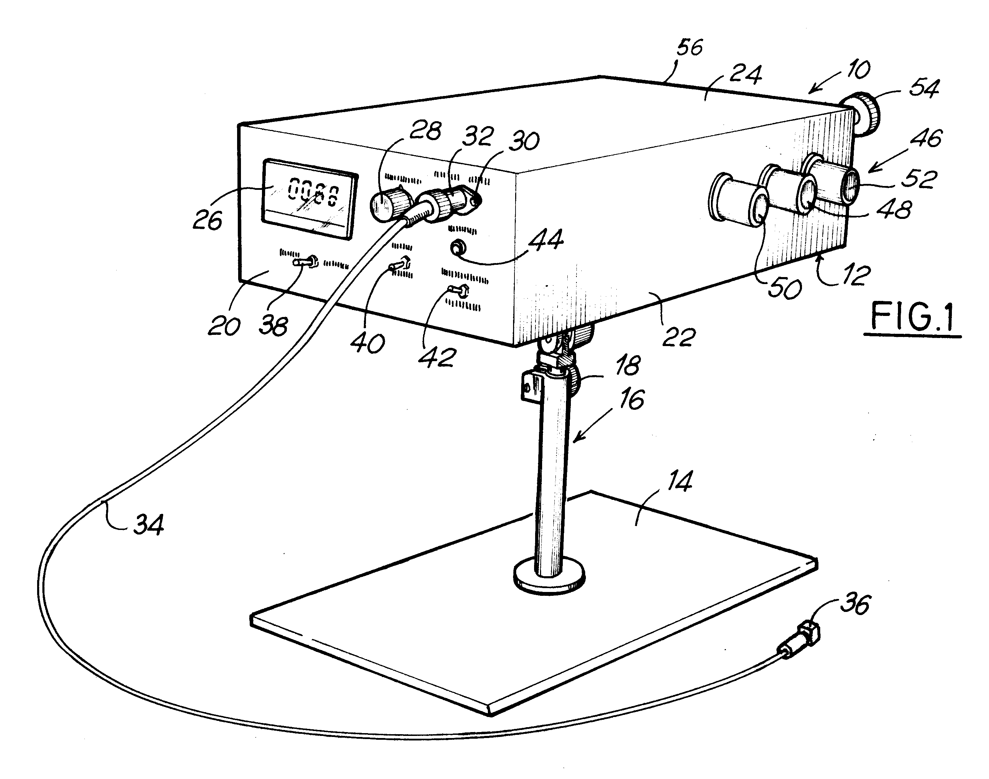

L'appareil de mesure 10 représenté à la figure 1 est essentiellement constitué d'un boîtier 12 de forme parallélépipédique rectangle qui est monté mobile réglable en hauteur par rapport à un socle 14 au moyen d'un pied télescopique 16 comportant une vis 18 de serrage du réglage en hauteur.

Parmi les différentes faces du boîtier 12, on distingue sur la figure la face avant 20, une face latérale 22 et la face supérieure 24.

La face avant 20 comporte un écran d'affichage digital 26 de la fréquence, un potentiomètre 28 pour le réglage manuel de la variation de la fréquence ainsi qu'une fiche 30 pour le raccordement d'une prise 32 fixée à l'extrémité d'un cordon de commande 34 dont l'autre extrémité est équipée d'un bouton de commande 36.

La face avant 20 comporte également un premier commutateur à deux positions 38 permettant de modifier la longueur d'onde d'émission de la source lumineuse intermittente et donc sa couleur, un deuxième commutateur à deux positions 40 permettant de passer d'une position manuelle de variation de la fréquence à une position de variation automatique et un troisième commutateur à deux positions 42 permettant de faire varier la fréquence dans le sens croissant ou décroissant.

La face avant 20 comporte enfin un bouton poussoir de réarmement ou de mise à zéro 44.

La face latérale 22 comporte les oculaires 46. Ces oculaires sont au nombre de trois et sont constitués par un premier oculaire central fixe 48 et par des deuxième et troisième oculaires 50 et 52 montés respectivement de part et d'autre de l'oculaire central 48 et de manière réglable comme cela sera expliqué plus avant à l'aide d'un bouton de réglage 54 situé sur la face arrière 56 du boîtier 12.

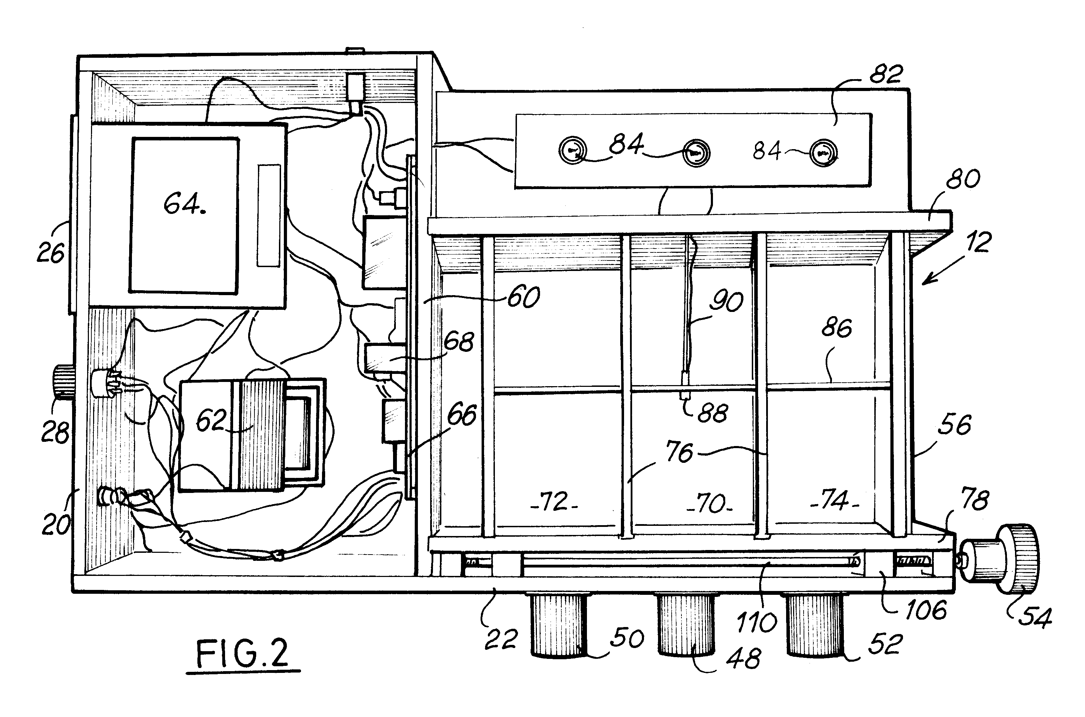

Dans la vue représentée à la figure 2, on a ôté la face supérieure 24 du boîtier 12 afin de faire apparaître les principaux éléments et composants qu'il contient.

La partie électronique de gauche, en considérant la figure 2, qui s'étend depuis la face avant 20 jusqu'à une cloison transversale intermédiaire 60 contient une source d'alimentation et de transformation électrique 62, un dispositif générateur de fréquence variable 64 et une plaque de circuit imprimé 66 sur laquelle sont montés divers composants électroniques 68 nécessaires au fonctionnement de l'appareil et qui permettent notamment l'enregistrement, ou mémorisation, et l'affichage de la fréquence de fusion sur l'écran 26.

La partie de droite, en considérant la figure 2, du boîtier 12 délimité par la cloison transversale 60 et par la face 56 du boîtier constitue la partie optique de l'appareil de mesure.

Cette partie du boîtier est divisée en trois couloirs parallèles 70, 72 et 74 qui sont chacun associés respectivement à l'oculaire 48, 50 et 52.

Les couloirs sont séparés par des cloisons transversales opaques parallèles 76.

Les cloisons 76 sont montées entre deux cloisons perpendiculaires 78 et 80 parallèles à la face latérale 22.

La première cloison 78 sert au montage réglable des oculaires, tandis que la seconde cloison 80 est transparente pour permettre l'éclairement des couloirs par un dispositif 82 comportant trois lampes à lumière blanche 84 qui ont pour but de créer dans les couloirs une lumière de fond sensiblement blanche perceptible par l'individu dont on va mesurer la fréquence de fusion. A cet effet, il est prévu une cloison en verre dépoli 86 qui créé un fond uniforme de lumière blanche.

La portion de la cloison 86 reçue dans le couloir central 70 permet le montage d'une diode électroluminescente 88 qui constitue la source lumineuse intermittente pour la mesure de la fréquence de fusion.

La diode 88 est reliée par une connexion électrique 90 à la partie électrique de gauche du boîtier 12.

Dans ce mode de réalisation de l'invention, la source lumineuse intermittente est une diode 88 (LED - PY5534S) qui peut émettre une lumière rouge ayant un pic d'émission d'une longueur d'onde de 660 nm ou une lumière verte ayant un pic d'émission d'une longueur d'onde de 555 nm.

L'intensité de la lumière émise par la diode 88 est maintenue constante par le circuit électronique d'une conception connue qui a également pour fonction d'alimenter la lampe de façon qu'elle émette de façon intermittente avec des impulsions de durée constante.

Les lumières rouge et verte sont préférées aux lumières bleue ou jaune clair du fait des défauts en réponse temporelle associés au mécanisme de détection de longueurs d'ondes courtes.

Une lumière rouge est perçue aisément et n'est pratiquement pas absorbée par la sclérose du cristallin et ceci de manière beaucoup plus efficace que dans le cas de longueurs d'ondes plus courtes.

La cloison 86 et les trois lampes 84, dont la puissance est pour chacune de six watts, produisent un fond illuminé homogène en arrière de la diode 88 qui est indépendant des conditions ambiantes d'éclairage autour de l'appareil dans la salle dans laquelle sont effectuées les mesures.

La diode LED 88 apparaît comme étant plus claire que le fond lumineux.

Afin de mesurer la fréquence de fusion de la fovéa de l'oeil, la diode 88 émet selon un arc de 1,6 degré, c'est-à-dire qu'elle constitue une source quasiment ponctuelle orientée en direction de l'oculaire 48.

Chacun des oculaires comporte une lentille de correction de huit dioptries de manière que l'image de la diode 88 soit à l'infini optique.

Il est donc nécessaire que l'individu dont on va mesurer la fréquence de fusion porte une correction optique correspondant à la correction optimale de sa vision de loin et qui sera d'un modèle uniforme pour tous les individus de manière à ne pas influencer la mesure d'un individu à l'autre en fonction par exemple du matériau constitutif de ses verres de correction.

Le générateur de fréquence 64 alimente la diode 88 en énergie à des intervalles égaux.

La fréquence d'émission peut être modifiée, selon un pas constant, de manière croissante ou décroissante automatiquement ou manuellement.

L'agencement des trois oculaires permet de réaliser pour chacun des yeux testés qui est en regard de l'oculaire central 48 un test par vision dite "binoculaire".

Les moyens de réglage du pied télescopique 16 permettent de régler la hauteur du boîtier 12 de manière que le plan qui contient les axes optiques des trois oculaires soit à la hauteur de la ligne passant par les axes pupillaires de l'individu.

Afin d'obtenir une mesure précise de la fréquence de fusion au clignotement de la fovéa de l'oeil, l'invention prévoit des moyens de réglage de l'écartement D des axes optiques des oculaires de manière à les adapter à l'écartement pupillaire de l'individu.

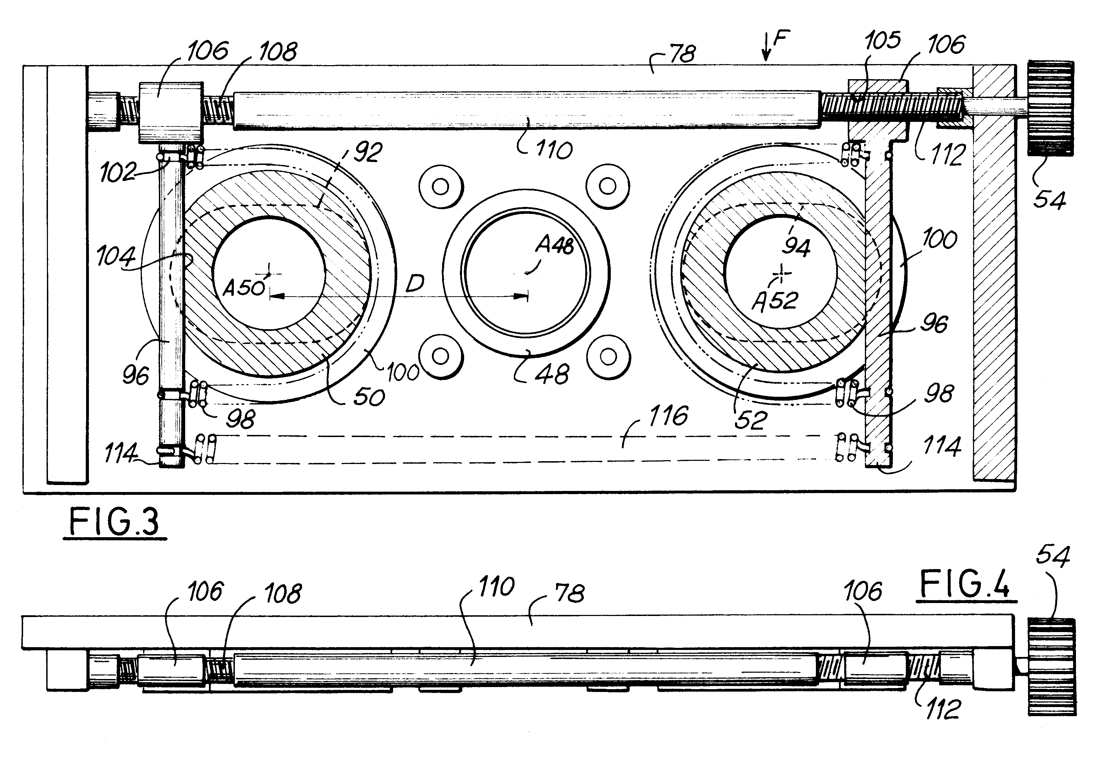

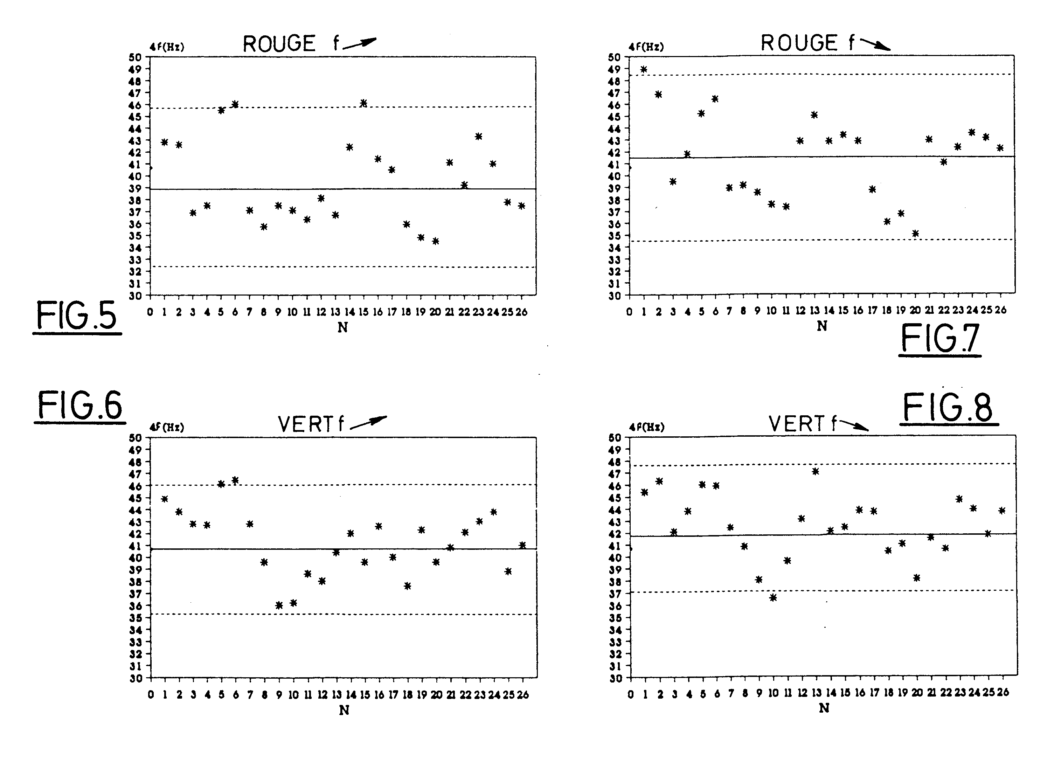

Ces moyens de réglage sont illustrés aux figures 3 et 4.

L'oculaire central 48 est fixe et son axe optique A48 est donc fixe par rapport à la cloison 78.

Les deux autres oculaires 50 et 52 sont réglables par rapport à l'oculaire 48 de manière que leurs axes optiques A50 et A52 puissent être simultanément rapprochés ou écartés de l'axe A48 de manière à faire varier la distance D et à l'adapter à l'écart pupillaire de l'utilisateur.

A cet effet, chacun des oculaires 50, 52 se déplace devant une fenêtre oblongue de la cloison 68, respectivement 92 et 94.

L'oculaire 50 est monté sur une potence 96 à l'aide d'un ressort 98 qui passe dans une gorge 100 de l'oculaire et dont les deux extrémités sont accrochées dans des rainures 102 de la potence.

La gorge 100 comporte un méplat 104 qui permet l'indexation et le positionnement de l'oculaire 50 par rapport à la potence 96.

L'extrémité supérieure 106 de la potence 96 est montée vissée sur une partie filetée 108 de la vis de réglage 110 doit la commande en rotation est assurée par le bouton 56.

Le troisième oculaire 52 est monté sur une potence identique à celle utilisée pour le deuxième oculaire 50 et pour la représentation de laquelle on a utilisé les mêmes chiffres de référence.

Pour la potence 96 associée à l'oculaire 52, le taraudage 105 de son extrémité supérieure 106 est monté vissé sur une portion filetée 112 de la vis de réglage 110.

Les sens des filetages des portions filetées 108 et 112 sont bien entendu inversés de manière à permettre un rapprochement, ou un éloignement, simultané des deux oculaires mobiles par rapport à l'oculaire fixe central 48 par l'entraînement en rotation de la vis 110.

Les extrémités inférieures 114 des potences 96 sont sollicitées élastiquement l'une vers l'autre par un ressort de rappel commun 116.

Du fait du montage des potences 96 entre la cloison 78 et la face latérale 22, elles sont bien entendu empêchées de tourner autour de l'axe de la vis de manière que la rotation de la vis 110 soit convertie en un mouvement de translation des potences 96.

La mesure de la fréquence de fusion de la fovéa de l'oeil d'un individu s'effectue de la manière suivante.

Après avoir mesuré l'écart pupillaire de l'individu, on règle la distance D entre les oculaires et l'on met le boîtier 12 à la bonne hauteur.

On commence par exemple par mesurer la fréquence de fusion de la fovéa de l'oeil droit de l'individu. A cet effet, l'individu se place devant les oculaires 50 et 48. On procède alors à la mesure de la fréquence de fusion de l'oeil droit en lumière rouge en commençant par faire varier la fréquence de clignotement de la diode 88 dans le sens croissant.

Selon une technique connue, l'individu presse sur le bouton de commande 36 lorsque la lumière émise par la diode 88 lui paraît devenir continue alors qu'il percevait initialement son clignotement.

L'action sur le bouton de commande 36 a pour but de provoquer l'enregistrement, par des moyens connus non décrits, de la fréquence de fusion de l'oeil droit en lumière rouge de fréquence F croissante.

On pratique ensuite à la mesure de la fréquence de fusion de la fovéa de l'oeil droit en lumière rouge de fréquence décroissante.

Sans changer de position on procède ensuite consécutivement à la mesure de la fréquence de fusion de la fovéa de l'oeil droit en lumière verte de fréquence croissante puis en lumière verte de fréquence décroissante.

L'individu change ensuite de position de manière que son oeil gauche soit devant l'oculaire fixe central 48 et l'on procède comme précédemment aux quatre mesures pour la fréquence de fusion de la fovéa de son oeil gauche.

Conformément à l'invention, l'utilisation de deux longueurs d'ondes d'émission pour la source lumineuse intermittente, correspondant aux lumières rouge et verte, permet d'éliminer le défaut d'évaluation de la fréquence de fusion dû à un défaut de perception des couleurs de l'individu.

Les graphiques des figures 5 à 8 représentent une campagne de mesures effectuées sur vingt-six individus selon la méthode décrite précédemment.

Sur chaque graphique, la ligne centrale en trait plein représente la fréquence de fusion moyenne et les deux lignes en pointillés illustrent l'écart type.

Le phénomène d'erreur dû à une mauvaise perception des couleurs est parfaitement visible sur ces graphiques si l'on considère par exemple l'individu identifié par son numéro d'ordre N = 3 pour lequel on constate que la fréquence de fusion en lumière verte, qu'elle soit mesurée en fréquence croissante ou décroissante, est nettement supérieure à sa fréquence de fusion mesurée en lumière rouge.

Dans le cas d'une telle disparité de mesures, on retient la fréquence de fusion la plus élevée obtenue, c'est-à-dire par exemple la fréquence de fusion mesurée en lumière verte pour l'individu n° 3.

L'appareil de mesure qui vient d'être décrit permet d'effectuer des mesures dans un temps très restreint. Il permet de maîtriser la totalité des paramètres qui ont une influence dans la mesure de la fréquence de fusion, telle que l'intensité de l'éclairement, l'aire de la portion de l'oeil qui est illuminé, la durée de l'exposition, l'état de la rétine et bien entendu la longueur d'onde ou la couleur de la source lumineuse intermittente.

Afin d'obtenir les meilleurs résultats possibles, il est souhaitable de prévoir une interruption de 30 secondes du test entre l'utilisation de la lumière rouge et de la lumière verte.

Une fois que l'appareil de mesure a été étalonné, il peut être utilisé très facilement par un médecin ou par des assistants.

La combinaison d'une source lumineuse ponctuelle et des différents moyens de réglage pour permettre d'adapter l'appareil à l'utilisateur permet de mesurer de manière très précise la fréquence de fusion de la fovéa de l'oeil.

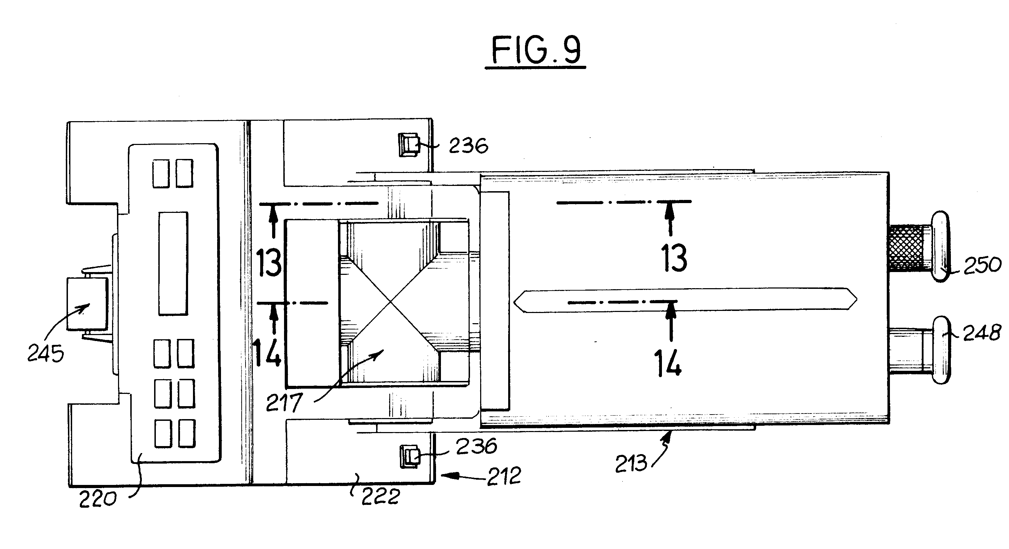

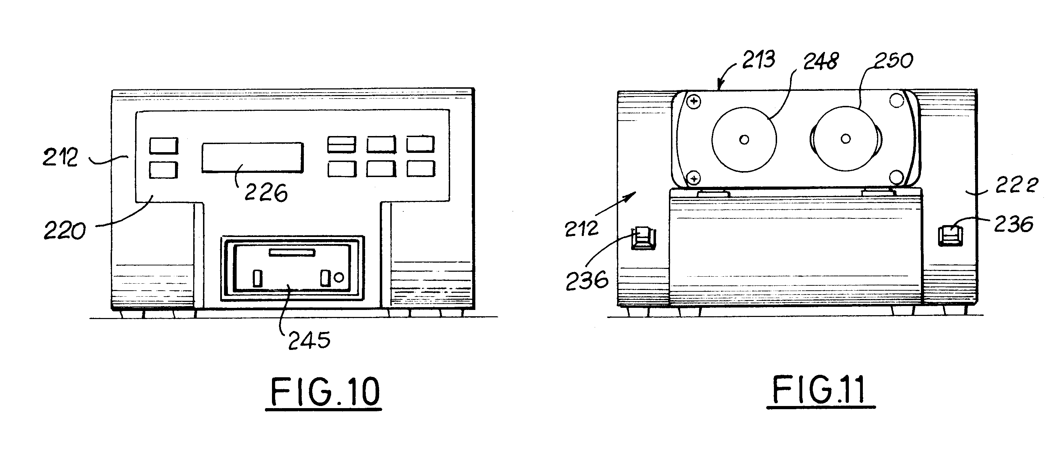

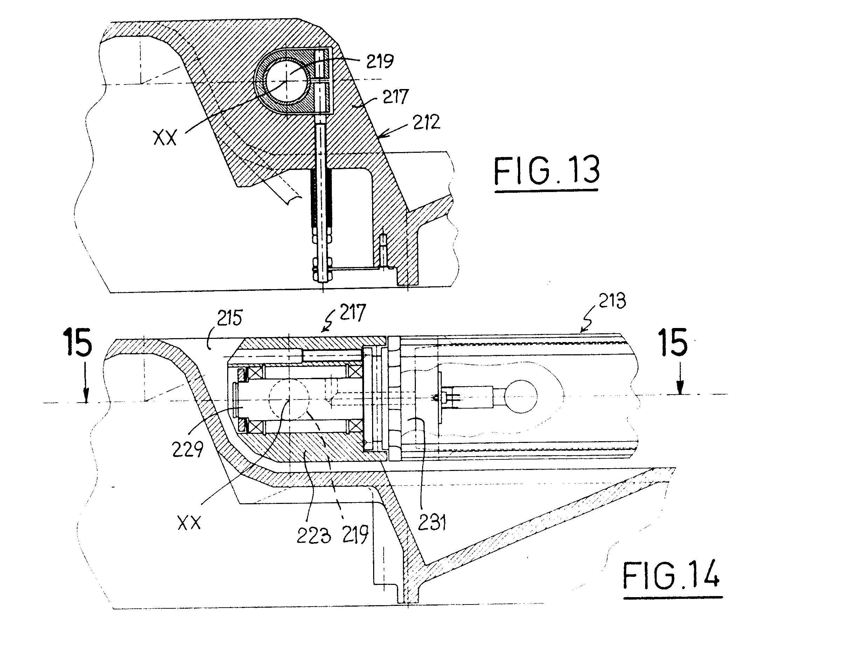

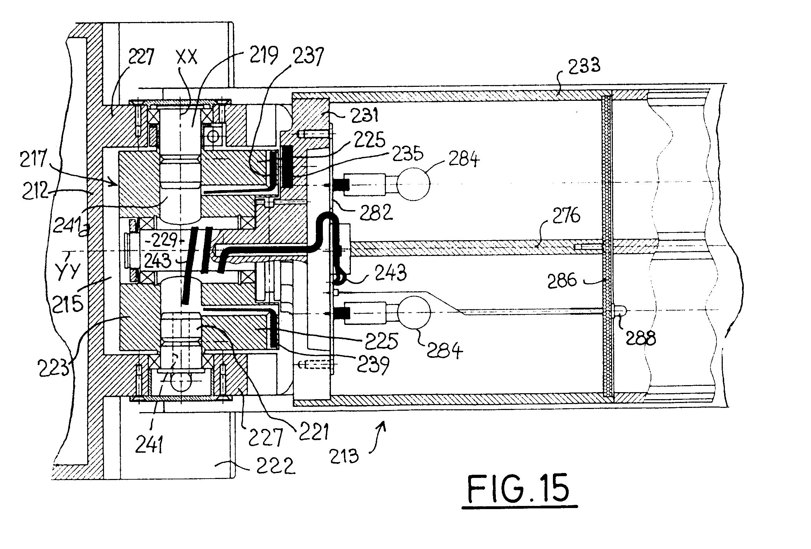

On décrira maintenant le mode de réalisation préféré de l'invention tel qu'il est illustré aux figures 9 à 15 et qui correspond à la version industrialisée de l'appareil qui vient d'être décrit.

Les éléments identiques ou équivalents seront désignés par les mêmes chiffres de référence augmentés de 200.

L'appareil est essentiellement constitué d'un boîtier 212 qui repose par exemple sur une table 211 et d'un groupe binoculaire articulé 213.

Le corps du boîtier 212 est creux et reçoit divers composants de l'appareil et notamment les circuits électroniques et d'alimentation électrique.

La partie centrale du boîtier 212 comporte un évidement central 215 dans lequel est reçue la portion d'extrémité 217 du groupe binoculaire 213 de manière articulée.

La partie 217 est articulée autour d'un axe horizontal X-X au moyen de deux tourillons 219 et 221 montés chacun d'une part dans une chape centrale 223 à deux bras 225 et d'autre part dans des parois 227 du boîtier.

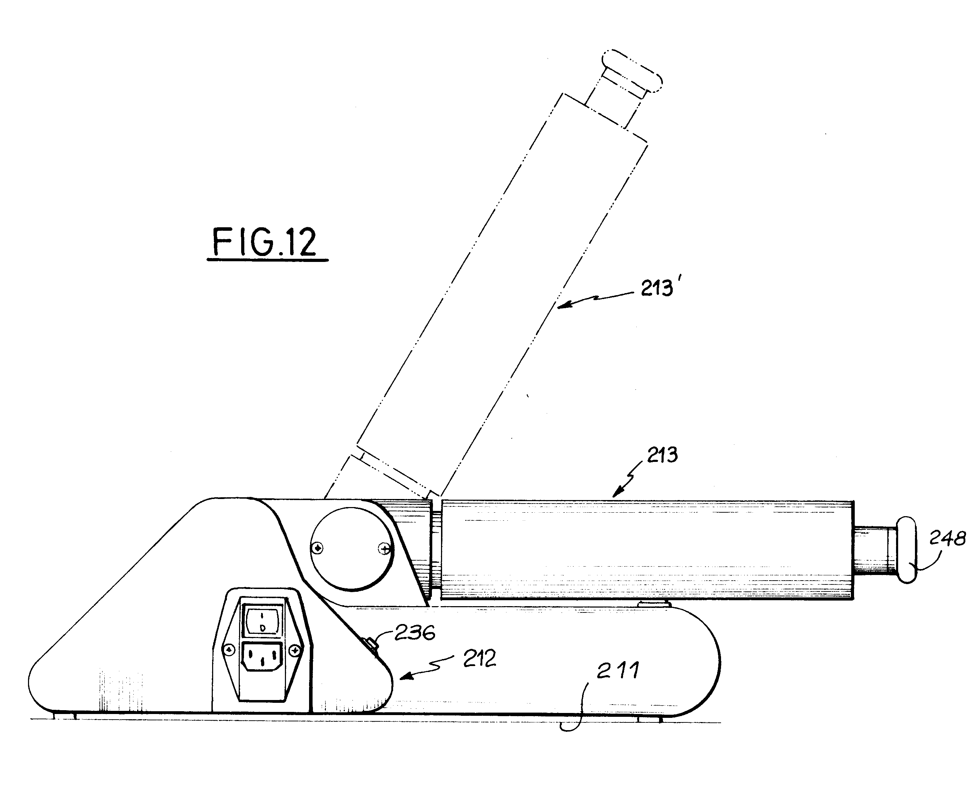

La partie 217 est ainsi montée pivotante autour de l'axe X-X pour permettre le pivotement du groupe binoculaire 213 autour de cet axe entre la position de repos illustrée en trait plein sur les figures et la position de mesure 213' illustrée en silhouette à la figure 12.

La chape centrale 223 reçoi également un pivot 229 relié à l'embase 231 du groupe binoculaire 213.

Le pivot est monté à rotation autour d'un axe Y-Y parallèle à la direction de visée optique et concourant et perpendiculaire à l'axe X-X, autour duquel le corps du binoculaire peut pivoter de 180°.

Le corps 233 du groupe binoculaire est séparé en deux parties par une cloison médiane 276 et transversalement par une cloison en verre dépoli 286 qui porte la source lumineuse 288.

L'embase 231 porte les lampes 284 au nombre de deux par l'intermédiaire d'une plaque 282.

La plaque 282 est raccordée à l'embase 231. Les contacts 237 et 239 de la chape 223 sont commandés par l'aimant permanent 235, fixé sur l'embase 231, selon la position angulaire du groupe 213 autour de l'axe Y-Y. Les contacts 237 et 239 sont reliés à l'alimentation par des conducteurs passant par un trou 241 formé dans l'axe 221 et par un trou 241a formé dans le bras 225.

L'alimentation des lampes 284 et de la source 288 est également assurée par des conducteurs 243 qui traversent le pivot 229 et passent par le trou 241.

L'oculaire 250 qui n'est pas situé en regard de la source 288 comporte des moyens de réglage optique et de sa position (écart D) par rapport à l'oculaire de mesure 248. Ces moyens n'ont pas été illustrés en détails sur les figures.

Le corps du boîtier 212 comporte sur sa face arrière 222 deux boutons de commande 236 agencés symétriquement à gauche et à droite et qui peuvent être actionnés indifféremment par le patient.

La face avant 220 du boîtier 212 comporte l'écran d'affichage digital 226, une imprimante 245 et différents boutons de commande permettant de choisir la couleur émise, le mode ascendant ou descendant de la fréquence, ainsi que différents modes de programmation de l'utilisation en vue notamment de son utilisation automatisée.

Il est par exemple possible d'associer à l'appareil de mesure tous les moyens de traitement informatisé connus à ce jour qui permettent de mémoriser automatiquement les valeurs relevées lorsque l'individu actionne le bouton de commande 236 en vue d'en faire une synthèse de manière automatique et à permettre notamment d'éliminer systématiquement le défaut dû à une mauvaise perception des couleurs.

De même l'ensemble des opérations successives aboutissant à la détermination de la fréquence de fusion peut être automatisé.

La source lumineuse peut être constituée de plusieurs diodes associées émettant chacune dans des couleurs différentes, mais il est important que la source demeure ponctuelle.

**Revendications**

1. Appareil (10) pour la mesure de la fréquence critique de fusion fovéale de l'oeil d'un individu, du type comportant :
   – au moins un oculaire (48) auquel l'individu applique son oeil;
   – une source lumineuse intermittente (88) visible par l'individu à travers l'oculaire (48);
   – des moyens (64) pour faire varier la fréquence d'émission de la source lumineuse intermittente (88);
   – des moyens d'enregistrement de la fréquence d'émission de la source lumineuse;
   – des moyens (36) de commande des moyens d'enregistrement déclenchés par l'individu lorsque la fréquence de fusion de l'oeil est atteinte; et
   – une source lumineuse (88) susceptible d'émettre selon plusieurs longueurs d'onde;
   caractérisé en ce que la source lumineuse émet selon au moins deux longueurs d'onde constantes correspondant à deux couleurs distinctes perceptibles par l'individu, et en ce que l'appareil comporte des moyens pour provoquer l'émission variable en fréquence de la source lumineuse aux deux longueurs d'ondes distinctes afin de mesurer successivement la fréquence critique de fusion fovéale de l'oeil de l'individu pour les deux longueurs d'ondes.

2. Appareil selon la revendication 1, caractérisé en ce que la source lumineuse comporte deux diodes électroluminescentes.

3. Appareil selon l'une des revendications 1 ou 2, caractérisé en ce que les couleurs distinctes sont le rouge et le vert.

4. Appareil selon l'une quelconque des revendications 1 à 3, caractérisé en ce que les moyens pour faire varier la fréquence permettent d'augmenter ou de réduire progressivement, selon un pas constant, la fréquence d'émission lumineuse pour chacune des deux longueurs d'ondes.

5. Appareil selon l'une quelconque des revendications 1 à 4, caractérisé en ce qu'il comporte un circuit électronique pour alimenter la source lumineuse (88) de manière que l'intensité de la lumière émise soit maintenue constante.

6. Appareil selon l'une quelconque des revendications 1 à 5, caractérisé en ce qu'il comporte un circuit électronique pour alimenter la source lumineuse (88) de manière qu'elle émette de façon intermittente avec des impulsions de durée constante au cours de la mesure.

7. Appareil selon l'une quelconque des revendications 1 à 6, caractérisé en ce qu'il comporte des moyens (84, 86) pour produire un fond illuminé homogène en arrière de la source lumineuse et perceptible par l'individu.

8. Appareil selon l'une quelconque des revendications 1 à 7, caractérisé en ce qu'il comporte un deuxième oculaire (50) et en ce que la source lumineuse (88) est visible par l'individu à travers un seul (48) des deux oculaires.

9. Appareil selon l'une quelconque des revendications précédentes pour la mesure de la fréquence de fusion, de la fovéa de l'oeil, caractérisé en ce que la source lumineuse (88) est sensiblement ponctuelle et en ce qu'il comporte des moyens de réglage de la position de l'oeil mesuré par rapport à la source lumineuse intermittente.

10. Appareil selon la revendication 8, caractérisé en ce que les deux oculaires (248, 250) font partie d'un groupe binoculaire (213) qui est monté articulé par rapport à un boîtier (212) de l'appareil autour de deux axes perpendiculaires et concourants (X-X, Y-Y) dont l'un (Y-Y) est parallèle à la direction de visée optique.

FIG.1

FIG.2

FIG.3

FIG.4

FIG.7

FIG.8

FIG.5

FIG.6

FIG. 9

FIG.11

FIG.10

12

FIG.12

EP 0 497 648 A1

FIG.13

FIG.14

14

FIG.15

**Office européen des brevets**

# RAPPORT DE RECHERCHE EUROPEENNE

Numero de la demande

EP    92 40 0125

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5 ) |
|---|---|---|---|
| D,X | WO-A-8 810 088 (J.C.DOWNING)<br>* le document en entier *<br>--- | 1,3,4 | A61B5/16<br>A61B3/02 |
| A | US-A-3 891 311 (J.C.FLETCHER ET AL.)<br>* colonne 3, ligne 4 - colonne 5, ligne 10 *<br>* colonne 29, ligne 7 - ligne 47; figures 1-10 *<br>--- | 1 | |
| A | US-A-4 324 460 (M.L.DALEY)<br>* le document en entier *<br>--- | 1 | |
| A | US-A-2 495 708 (R.H.DRAEGER ET AL.)<br>* le document en entier *<br>--- | 1 | |
| A | US-A-2 057 983 (H.R.SPITLER ET AL.)<br>* le document en entier *<br>--- | 1 | |
| A | US-A-4 740 072 (J.P.GRIFFIN ET AL.)<br>* abrégé; figures 1-11 *<br><br>----- | 1 | |

**DOMAINES TECHNIQUES RECHERCHES (Int. Cl.5 )**

A61B

Le présent rapport a été établi pour toutes les revendications

| Lieu de la recherche | Date d'achèvement de la recherche | Examinateur |
|---|---|---|
| LA HAYE | 11 MAI 1992 | HUNT B.W. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)